(19) Europäisches Patentamt
European Patent Office
Office européen des brevets

(11) **EP 4 242 580 A1**

(12) **EUROPEAN PATENT APPLICATION**

(43) Date of publication:
**13.09.2023  Bulletin 2023/37**

(21) Application number: **22161221.1**

(22) Date of filing: **09.03.2022**

(51) International Patent Classification (IPC):
***G01B 9/02091*** *(2022.01)*

(52) Cooperative Patent Classification (CPC):
**G01B 9/02091; A61B 5/0066; G01B 9/02044;
G01B 9/02083**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **National University of Ireland Galway
Galway (IE)**

(72) Inventors:
• **Alexandrov, Sergey
Galway (IE)**

• **McAuley, Ryan
Galway (IE)**
• **Leahy, Martin
Limerick, V94 EHH2 (IE)**
• **Nolan, Andrew
Dublin 10 (IE)**

(74) Representative: **Barker Brettell LLP
100 Hagley Road
Edgbaston
Birmingham B16 8QQ (GB)**

(54) **IMAGING APPARATUS AND METHOD**

(57)    There is provided an apparatus (300) for imaging an object. The apparatus (300) comprises an imaging system (310) configured to obtain a spectrum comprising one or more axial spatial frequencies representing at least a part of the object. The apparatus (300) also comprises a processor (312). The processor (312) is configured to synthesize or generate a spatial harmonic corresponding to each spatial frequency in the spectrum. The processor (312) is also configured to reconstruct an image of a depth profile of the at least a part of the object using the spatial harmonics.

**EP 4 242 580 A1**

100 ⟶

102 | Obtain spectrum of axial spatial frequencies for object

↓

104 | Produce synthetic spectrum including corresponding negative frequencies

↓

106 | Reconstruct image of object from synthetic spectrum

FIGURE 3

**Description**

TECHNICAL FIELD

The present invention relates to an apparatus for and method of imaging an object, and in particular but not exclusively to an apparatus for and method of imaging an object using optical coherence tomography.

BACKGROUND

Optical Coherence Tomography (OCT) is a fast-developing imaging modality to form depth resolved images of scattering objects.

OCT has revolutionised medical diagnostics in a number of specialties, most notably in ophthalmology.

OCT occupies a major share (over 70%) of the total optical imaging market worldwide. The global OCT market is also anticipated to continue growing rapidly, reaching a value of 1.4 billion USD by the year 2023.

However, depth resolution of OCT imaging is limited by the spectral bandwidth of the light source, and typically is around 5 to 10 $\mu$m in air. That depth resolution is not suitable for many applications, including visualisation and analysis of small morphological features in biological tissue - for example, for early detection of different pathologies, including cancer.

The straightforward approach to improve axial or depth resolution of OCT images is to use a light source with shorter wavelengths and a broader spectral bandwidth. For example, Ti: Sapphire lasers, super-continuum lasers and thermal light sources have been used to obtain around 1$\mu$m axial resolution in biological tissue, using a spectral range from visible light to 1105nm [1-3]. However, that approach is impractical, cumbersome and adds complexity such as dispersion.

Other techniques to improve the axial resolution at a given spectral bandwidth, usually referred to as super-resolution OCT, have been attempted. Those techniques include de-convolution methods [4, 5], different sampling methods in the K-space [6], obtaining super-resolved OCT images from recording multiple lower resolution images [7], spectral estimation OCT to avoid digital inverse Fourier transform [8, 9], modelling of OCT images by the scale mixture models [10], and generative adversarial network-based techniques [11]. Nano-sensitive OCT has also been developed to improve sensitivity to structural changes [12-20].

However, attempts to extract more information from the limited spectral bandwidth of the OCT signal require further work to improve the axial or depth resolution of OCT imaging.

The present invention has been devised with the foregoing in mind.

SUMMARY

According to a first aspect of the invention, there is provided an apparatus for imaging an object. The apparatus may comprise an imaging system. The imaging system may be configured to obtain a spectrum comprising one or more axial spatial frequencies representing at least a part of the object. The apparatus may further comprise a processor. The processor may be configured to produce a synthetic spectrum. The synthetic spectrum may comprise the axial spatial frequencies, and a corresponding negative frequency for each of the axial spatial frequencies. The processor may be further configured to reconstruct an image of a depth profile of the at least a part of the object from the synthetic spectrum.

By reconstructing an image of the depth profile using a synthetic spectrum comprising both the axial spatial frequencies and corresponding negative frequencies, fine structure of an object that cannot be resolved using conventional OCT or optical coherence microscopy (OCM) imaging may be resolved in the reconstructed image. A depth resolution of an image reconstructed using a synthetic spectrum may be smaller than a resolution limit of the imaging system used. For example, the depth resolution may be substantially 10 times smaller than for conventional OCT imaging. That approach may also allow quantitative access to sub-wavelength structure within regions/volumes of interests smaller, than resolution limit of the imaging system, and nanoscale sensitivity to structural changes.

The processor may be configured to reconstruct the image of the depth profile of the at least a part of the object with a depth resolution smaller than a resolution limit of the imaging system. The reconstructed image may have a depth resolution of R $\approx \lambda_{min}/2n$, where R is the resolution, $\lambda_{min}$ is the minimum wavelength of the spectrum used to image the object, and n is the refractive index of the object.

The imaging system may be or comprise an OCT or OCM imaging system. The OCT or OCM imaging system may be or comprise a Fourier domain OCT or OCM imaging system, for example a spectral domain OCT imaging system (SDOCT) or a swept source OCT imaging system (SSOCT).

The processor may be configured to reconstruct the image of the depth profile of the at least a part of the object by performing an inverse Fourier transform of the synthetic spectrum.

The processor may be configured to represent the synthetic spectrum along an axial direction of the object as:

$$U(v_Z) = \begin{cases} U_1(|v_Z|), for\ v_{Zmin} \leq v_z \leq v_{Zmax}, -v_{Zmax} \leq v_z \leq -v_{Zmin} \\ I_0, for\ v_z = 0 \\ 0, for\ the\ rest \end{cases}$$

The spectrum may comprise both one or more axial spatial frequencies and one or more lateral spatial frequencies. The spectrum may be a 2D or 3D spectrum. The processor may be configured to produce a synthetic spectrum comprising the axial spatial frequencies and lateral spatial frequencies, and corresponding negative frequencies for each of the axial spatial frequencies and lateral spatial frequencies. The synthetic spectrum may be a 2D or 3D synthetic spectrum.

The processor may be configured to determine quantitative information about the object. The processor may be configured to determine the quantitative information about the object with a substantially nanoscale sensitivity.

The processor may be configured to generate a map showing a distribution of the determined quantitative information about the object. The processor may be configured to generate the map by displaying each point in the reconstructed image using a parameter value (e.g., a colour or a shade) corresponding to the quantitative information for that point in the image. The generated map may have a depth resolution smaller than a resolution of the imaging system used. That approach may enable depth-resolved visualization and quantitative measurement or estimation of sub-wavelength internal structure of an object, with both nanoscale sensitivity and depth resolution below the resolution limit of the imaging system used.

The processor may be configured to determine quantitative information about the object from the reconstructed image. The processor may be configured to determine quantitative information about one or more resolved structures in the reconstructed image. The processor may be configured to determine an axial spatial period of the object at each point in the reconstructed image. The processor may be configured to generate a map of axial spatial period distribution of the object. The processor may be configured to determine the quantitative information about the structure of the object with a substantially nano-scale sensitivity.

The processor may alternatively or additionally be configured to determine quantitative information about the object from the synthetic spectrum. The processor may be configured to determine an axial spatial frequency profile at each point in the reconstructed image. The processor may be configured to generate a map based on one or more parameters of the axial spatial frequency profiles, for example a maximum spatial period or a mean spatial period, or a correlation between spatial period profiles etc.

According to a second aspect, there is provided a method of imaging an object. The method may be computer-implemented. The method may comprise obtaining a spectrum comprising one or more axial spatial frequencies representing at least a part of the object. The method may also comprise producing a synthetic spectrum. The synthetic spectrum may comprise the axial spatial frequencies and a corresponding negative frequency for each of the axial spatial frequencies. The method may further comprise reconstructing an image of a depth profile of the at least a part of the object from the synthetic spectrum.

The method of the second aspect may be performed on or using the apparatus of the first aspect. The method of second aspect may comprise one or more optional features corresponding to optional features of the apparatus of the first aspect.

The method may comprise reconstructing the image of the depth profile of the at least a part of the object having a depth resolution smaller than a resolution limit of the imaging system.

Obtaining the spectrum comprising the one or more axial spatial frequencies representing the at least a part of the object may comprise using optical coherence tomography (OCT), for example Fourier domain OCT such as spectral domain OCT (SDOCT) or swept source OCT (SSOCT).

The spectrum may comprise the one or more axial spatial frequencies and one or more lateral spatial frequencies of the object. The spectrum may be a 2D or 3D spectrum. The synthetic spectrum may comprise the axial spatial frequencies and the lateral spatial frequencies, and corresponding negative frequencies for each of the axial spatial frequencies and lateral spatial frequencies. The synthetic spectrum may be a 2D or 3D synthetic spectrum.

Reconstructing the image of the depth profile of the at least a part of the object may comprise performing an inverse Fourier transform of the synthetic spectrum.

The synthetic spectrum along an axial direction of the object may be represented as:

$$U(v_Z) = \begin{cases} U_1(|v_Z|), for\ v_{Zmin} \leq v_z \leq v_{Zmax}, -v_{Zmax} \leq v_z \leq -v_{Zmin} \\ I_0, for\ v_z = 0 \\ 0, for\ the\ rest \end{cases}$$

The method may comprise determining quantitative information about the object. The method may comprise determining quantitative information about the object with a substantially nano-scale sensitivity.

The method may comprise generating a map showing a distribution of the determined quantitative information about the object. The method may comprise generating the map by displaying each point in the reconstructed image using a parameter value (e.g., a colour or a shade) corresponding to the quantitative information for that point in the image. The generated map may have a depth resolution smaller than a resolution of the imaging system used. That may enable depth-resolved visualization and quantitative measurement or estimation of sub-wavelength internal structure of an object, with both nanoscale sensitivity and depth resolution below the resolution of the imaging system used.

The method may comprise determining quantitative information about the object from the reconstructed image. The method may comprise determining quantitative information about one or more resolved structures in the reconstructed image. The method may comprise determining an axial spatial period of the object at each point in the reconstructed image. The method may comprise generating a map of axial spatial period distribution of the object. The method may comprise determining the quantitative information about the structure of the object with a substantially nano-scale sensitivity.

The method may alternatively or additionally comprise determining quantitative information about the object from the synthetic spectrum. The method may comprise determining an axial spatial frequency profile at each point in the reconstructed image. The method may comprise generating a map based on one or more parameters of the axial spatial frequency profiles, for example a maximum spatial period or a mean spatial period, or a correlation between spatial period profiles etc.

According to a third aspect, there is provided a non-transitory computer program comprising instructions which, when the program is executed by a processor, cause the processor carry out the method of second aspect.

According to a fourth aspect, there is provided a computer-readable medium having the computer program of the third aspect stored thereon.

According to a fifth aspect of the invention, there is provided an apparatus for imaging an object. The apparatus may comprise an imaging system. The imaging system may be configured to obtain a spectrum comprising one or more axial spatial frequencies representing at least a part of the object. The apparatus may further comprise a processor. The processor may be configured to synthesize or generate a spatial harmonic corresponding to each spatial frequency in the spectrum. The processor may also be configured to reconstruct an image of a depth profile of the at least a part of the object using the spatial harmonics.

The processor may be configured to reconstruct the image of the depth profile of the at least a part of the object by superposition of the spatial harmonics.

The processor may be configured to synthesize the spatial harmonics and reconstruct the image of the depth profile of the at least a part of the object by producing a synthetic spectrum comprising the axial spatial frequencies and a corresponding negative frequency for each of the axial spatial frequencies, and reconstructing the image of the depth profile of the at least a part of the object from the synthetic spectrum.

The processor may be configured to reconstruct the image of the depth profile of the at least a part of the object by performing an inverse Fourier transform of the synthetic spectrum.

Features which are described in the context of separate aspects and embodiments of the invention may be used together and/or be interchangeable wherever possible. Similarly, where features are described in the context of a single embodiment for brevity, those features may also be provided separately or in any sub-combination. Features described in connection with the apparatus of the first aspect or the apparatus of the fifth aspect may have corresponding features definable with respect to the method of the second aspect, computer program of the third aspect or computer-readable medium of the fourth aspect, and vice versa, and those embodiments are specifically envisaged.

BRIEF DESCRIPTION OF THE DRAWINGS

The invention will now be described, by way of example only, with reference to the accompanying drawings in which:

FIGs. 1A to 1D show conventional OCT imaging;

FIGs. 2A to 2D show synthetic OCT imaging in accordance with an embodiment of the invention;

FIG. 3 shows a method of imaging an object using synthetic OCT imaging in accordance with an embodiment of the invention;

FIG. 4 shows another method of imaging an object using synthetic OCT imaging in accordance with an embodiment of the invention;

FIGs. 5A to 5I show an interferogram, a synthetic spectrum produced using the interferogram, an A-scan formed from the synthetic spectrum, an adjusted A-scan, and a B-scan showing magnified portions reconstructed using both i) conventional OCT imaging and ii) synthetic OCT imaging in accordance with an embodiment of the invention;

FIG. 6 shows an apparatus for imaging an object using synthetic OCT imaging in accordance with an embodiment of the invention;

FIGs. 7A to 7E show images of two experimental samples having a known internal periodic structure reconstructed using both i) conventional OCT imaging and ii) synthetic OCT imaging in accordance with an embodiment of the invention;

FIGs. 8A to 8E shows images of a glass plate reconstructed using both i) conventional OCT imaging and ii) synthetic OCT imaging in accordance with an embodiment of the invention, and an A-scan representing the point spread functions for both i) conventional OCT imaging and ii) synthetic OCT imaging in accordance with an embodiment of the invention;

FIGs. 9A to 9C show images of an air gap between two glass plates formed using i) optical microscopy, ii) conventional OCT imaging and iii) synthetic OCT imaging in accordance with an embodiment of the invention;

FIG. 10A to 10G show an image of a cucumber formed using conventional OCT imaging, with magnified portions reconstructed using both i) conventional OCT imaging and ii) synthetic OCT imaging in accordance with an embodiment of the invention;

FIG. 11 shows images of a human finger in vivo, with a number of magnified portions reconstructed using both i) conventional OCT imaging and ii) synthetic OCT imaging in accordance with an embodiment of the invention;

FIGs. 12A and 12B show images of the two experimental samples shown in FIGs. 7A to 7E, and magnified portions reconstructed using colour synthetic OCT imaging in accordance with an embodiment of the invention; and

FIGs. 13A to 13D shows a conventional OCT image of a slice of a potato, with magnified portions reconstructed using i) conventional OCT imaging, ii) colour synthetic OCT imaging in accordance with an embodiment of the invention, and iii) nanosensitive colour OCT imaging.

Like reference numbers and designations in the various drawings indicate like elements.

DETAILED DESCRIPTION

It is known that an object can be represented as a three-dimensional Fourier transform of spatial frequencies. Physically, these spatial frequencies correspond to or can be represented as spatial harmonics (for example, spatial, harmonically varying refractive indices) with different periodicities, amplitudes, phases and orientations within an object. The object can be reconstructed as a superposition of those spatial harmonics.

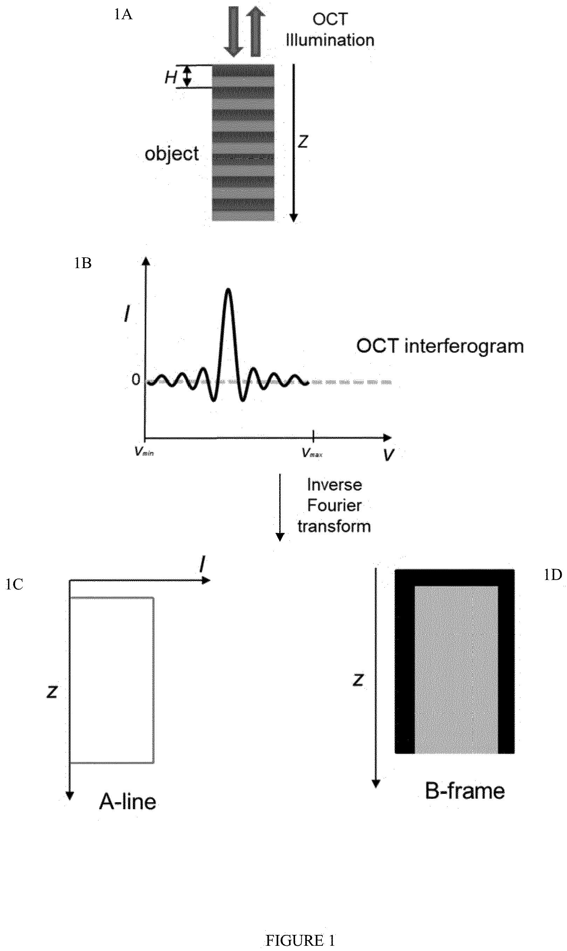

Figures 1A to 1D show conventional OCT imaging of an object. Figure 1A shows an example of an object having a spatial period H (formed by a harmonic, spatially oscillating refractive index depth profile, with adjacent regions having different refractive indices), and Figure 1B shows an OCT signal (for example, OCT interferogram) formed by detecting light scattered by a single spatial frequency $v$ of the object (corresponding to the spatial period H). OCT typically works in a reflection configuration, so the OCT signal is formed by light scattered at small, sub-wavelength size structures and information about high axial spatial frequencies of the object is present in the OCT signal. The coherence transfer function of OCT is symmetrical relative to lateral spatial frequencies but shifted to high axial spatial frequencies [26]. In the example shown, the spatial frequency $v$ is within the bandwidth of spatial frequencies detected using the given OCT system, and the OCT signal is formed by scattering at a small, sub-micron sized structure with spatial period H (corresponding to a high spatial frequency in the Fourier domain [12], where the frequency $v$ is inversely proportional to the spatial period H).

However, using conventional image reconstruction, because this period of structure is significantly smaller than the resolution limit of the OCT system, the corresponding spatial harmonic cannot be reconstructed and the fine structure of the spatial period H cannot be resolved. After reconstruction of the depth profile, for example using an inverse Fourier transform, information about the sub-micron sized structure is lost, as shown in Figures 1C (A-line or A-scan, showing a one-dimensional plot of amplitude or intensity I as a function of depth z) and 1D (B-frame or B-scan, showing a two-dimensional reconstructed image). A B-frame or B-scan may be reconstructed using a plurality of A-lines or A-scans obtained for different parts of the object.

The reason for that is, according to the shift theorem, the absolute value of the Fourier transform does not depend on the location (i.e., frequency) of the OCT signal in the Fourier domain. The reconstructed depth profile will be the same whether the OCT signal is located in a high spatial frequency region or a low spatial frequency region of the Fourier domain.

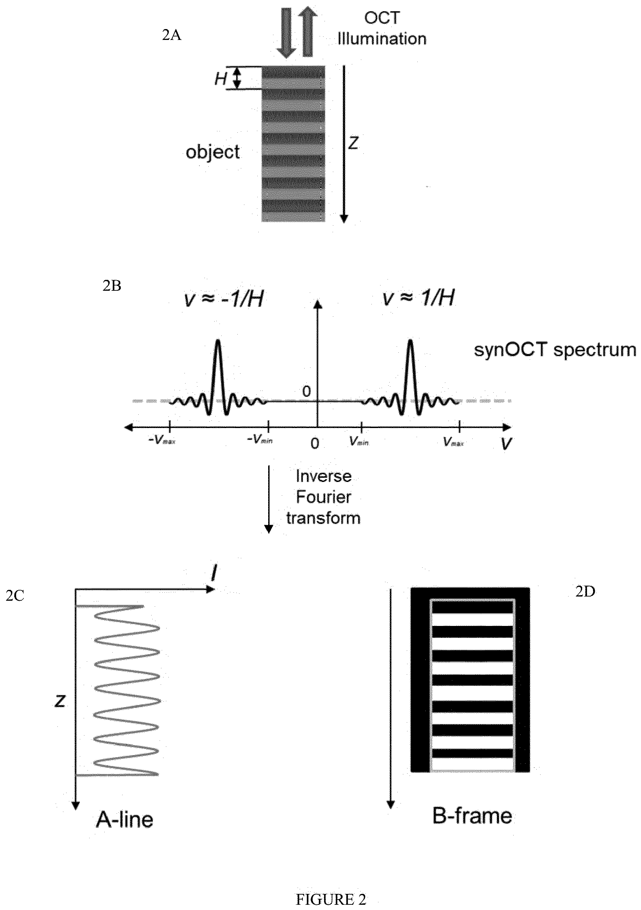

Figures 2A to 2D shows OCT imaging of an object in accordance with an embodiment of the invention. Figure 2A shows the same object as shown in Figure 1A, having the same spatial period H. Figure 2B shows an OCT signal formed by detecting light scattered by a structure of the object with a single spatial frequency $v$ (corresponding to the spatial period H). However, in Figure 2B, the OCT signal is modified to also include a negative frequency -v, corresponding to the detected spatial frequency v. In that way, a synthetic OCT signal or spectrum comprising the spatial frequency $v$ and the corresponding negative frequency -v is formed.

The depth profile is reconstructed using an inverse Fourier transform on the synthetic spectrum shown in Figure 2C. That approach means the spatial harmonic can be reconstructed and the fine structure of the spatial frequency $v$ can be seen in the reconstructed image, as shown in Figures 2C (A-line or A-scan) and 2D (B-frame or B-scan).

The same approach (referred to herein as synthetic OCT imaging or synOCT for convenience) can be used for all other spatial frequencies detected using OCT imaging, in order to reconstruct the fine structure of the object as a whole. In an example, the synthesized Fourier spectrum of the object may be written as

$$U(v_z) = \begin{cases} U_1(|v_z|), \; for \; v_{zmin} \leq v_z \leq v_{zmax} \, , \; -v_{zmax} \leq v_z \leq -v_{zmin} \\ I_0 \, , \quad for \; v_z = 0 \\ 0, \; for \; the \; rest \end{cases} \qquad (1)$$

where $U_1(|v_z|)$ is the collected OCT signal, converted into spatial frequencies [18], $v_{zmin}$ and $v_{zmax}$ are the minimum and maximum detected spatial frequencies, and $I_0$ is the constant which determines the DC level.

The Fourier spectrum of each spatial harmonic within an object has two components - a positive and a negative component. To reconstruct such a spatial harmonic, both the positive and negative components are required. In conventional OCT imaging, only one spatial frequency component of the Fourier spectrum is detected, meaning the spatial harmonic cannot be reconstructed.

Using the synOCT approach, the OCT signal (converted into a spectrum of spatial frequencies) will be fixed at the proper region of the Fourier domain, with both the positive and negative components present in the synthetic spectrum, allowing the spatial harmonics and the fine structure of the object to be reconstructed using an inverse Fourier transform

$$I(z) = F^{-1}[U(v_z)] \qquad (2)$$

As a result, high spatial frequency information is present in the reconstructed image rather than being lost. For an object comprising complex structures consisting of many spatial frequency components, the synOCT approach may enable reconstruction of all the spatial harmonics and an image to be formed. For example, an inverse Fourier transform of the synthetic spectrum may result in an image being formed as a superposition of all spatial frequency harmonics over a given bandwidth of illumination, showing the fine structure of the object.

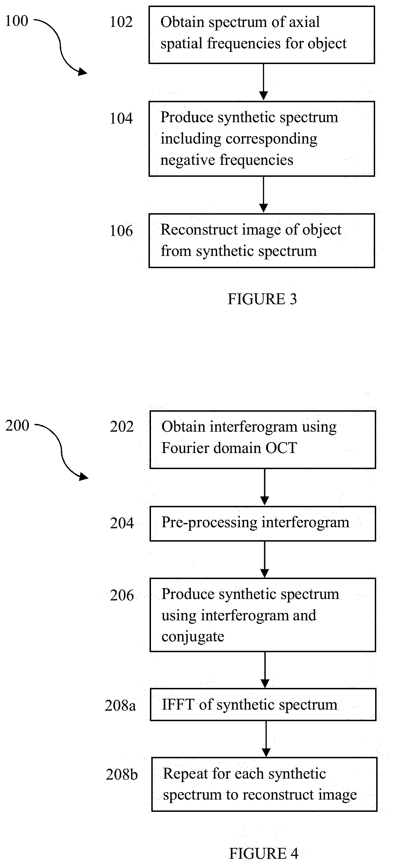

Figure 3 shows a method 100 for imaging an object, in accordance with an embodiment of the invention.

Step 102 of the method 100 comprises obtaining a spectrum comprising one or more axial spatial frequencies representing at least a part of the object. Obtaining the spectrum of axial spatial frequencies may comprise using OCT (for example, either swept source OCT (SSOCT) or spectral domain OCT (SDOCT)), although that is not essential.

Step 104 of the method 100 comprises producing a synthetic spectrum comprising the axial spatial frequencies representing the at least a part of the object, and a corresponding negative frequency for each of the axial spatial frequencies.

Step 106 of the method 100 comprises reconstructing an image of a depth profile of the at least a part of the object from the synthetic spectrum. Reconstructing the image of the depth profile may comprise performing an inverse Fourier transform of the synthetic spectrum, although that is not essential.

Figure 4 shows another method 200 for imaging an object, in accordance with an embodiment of the invention.

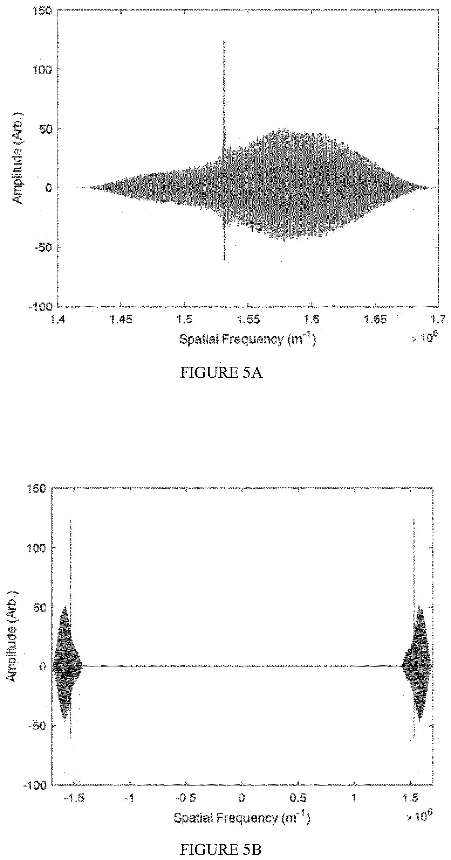

Step 202 of the method 200 comprises obtaining a spectrum comprising one or more axial spatial frequencies representing at least a part of the object. In the embodiment shown, the spectrum is an interferogram obtained using a Fourier domain OCT system (SSOCT or SDOCT), as shown in Figure 5A, although that is not essential. The peak corresponding to the dominant spatial frequency of the object can be clearly seen. The spectrum may alternatively be obtained using a different technique, for example time domain OCT.

Step 204 of the method 200 comprises pre-processing the obtained spectrum, although that is not essential.

Pre-processing of the interferogram may comprise the following conventional steps:

1. Calibration of the detected spectrum
2. Dispersion compensation
3. DC subtraction
4. Apodization
5. K-space linearization

The pre-processing steps described above may be carried out in any suitable order. Alternatively, only some of the pre-processing steps described above may be carried out. It will also be appreciated additional pre-processing steps may be carried out on the spectrum.

Step 206 of the method 200 comprises producing a synthetic spectrum comprising the axial spatial frequencies representing the at least a part of the object, and a corresponding negative frequency for each of the axial spatial frequencies. In the embodiment shown, producing the synthetic spectrum comprises placing the interferogram at the correct frequency range in the Fourier domain, and placing a conjugate interferogram at the corresponding negative frequency range, as shown in Figure 5B.

The synthetic spectrum may be zero padded to realise different sampling spacing along the depth profile. For example, at points where the signal intensity is at or below a threshold (for example, a predetermined threshold), a noise level or zero intensity, the signal may be considered as equal to 0. All points located between the positive interferogram and the conjugate negative interferogram therefore have a value of 0. In addition, 0 values may be used for points either side of the positive interferogram and the conjugate negative interferogram. That may enable the synthetic spectrum to be extended in both the positive and negative directions. By doing that, a sampling (distance between points in the depth profile) of the reconstructed image may be controlled. For example, a broader spectrum may result in a smaller distance between points in the reconstructed image. To obtain a reconstructed image having a desired resolution, there should be at least some points located within the resolved interval in depth profile. For that the synthetic spectrum may be extended using zero padding.

In some embodiments, producing the synthetic spectrum may comprise producing a synthetic spectrum from a 2D or 3D spectrum of spatial frequencies, including both axial and lateral spatial frequencies. The lateral spatial frequencies of the 2D or 3D spectrum may be obtained by lateral scanning along the object. A Fourier transform may then be performed along the lateral coordinates, optionally followed by resampling. Alternatively, wide-field imaging may be used to obtain lateral spatial frequencies.

The resulting spectrum may then be formed or rescaled, for example according to general scattering theory (such as along caps of Ewald spheres in the Fourier domain [26]). The 2D or 3D synthetic spectrum of spatial frequencies may subsequently be formed by adding the negative part of the spectrum, substantially as described above.

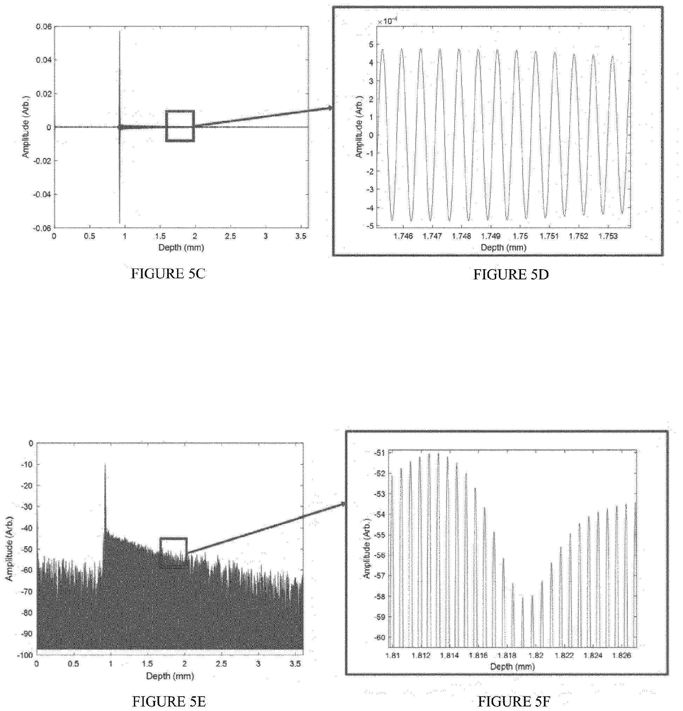

Step 208 of the method 200 comprises reconstructing an image of a depth profile of the at least a part of the object from the synthetic spectrum. In the embodiment shown, reconstructing the image of the depth profile comprises, at step 208a, performing an inverse Fourier transform (e.g., an inverse FFT) of the synthetic spectrum to form an A-line or A-scan, as shown in Figures 5C and 5D. Figure 5D shows a magnified portion of a selected region of the A-line shown in Figure 5C. Depending on the imaging needs, the A-line may be adjusted, although that is not essential. For example, parts of the A-line having an amplitude or intensity less than or equal to 0 may be removed from the A-line, with the resulting adjusted A-line plotted on a dB scale, as shown in Figures 5E and 5F. Figure 5F shows a magnified portion of a selected region of the adjusted A-line shown in Figure 5E.

At step 208b, the method 200 comprises repeating the step of 208a for at least a portion of the B-frame or B-scan (e.g., using a plurality of A-lines or A-scans obtained for different parts of the object), to form a reconstructed two-dimensional image of the depth profile of the object. A plurality of A-lines or A-scans obtained in directions perpendicular to one another may be used to reconstruct a 3D image. For example, using a plurality of parallel A-scans in the X direction a B-scan can be formed. To obtain a 3D image scans must also be performed in the perpendicular Y direction. Therefore, each point along the X direction will have a B-scan along the Y direction. It will be appreciated step 208b may not be necessary, for example if the synthetic spectrum is or comprises a 2D or 3D spectrum, as described above. In such a case, reconstruction of a 2D or 3D image may be performed by performing an inverse Fourier transform of the synthetic spectrum as described above with respect to step 208a.

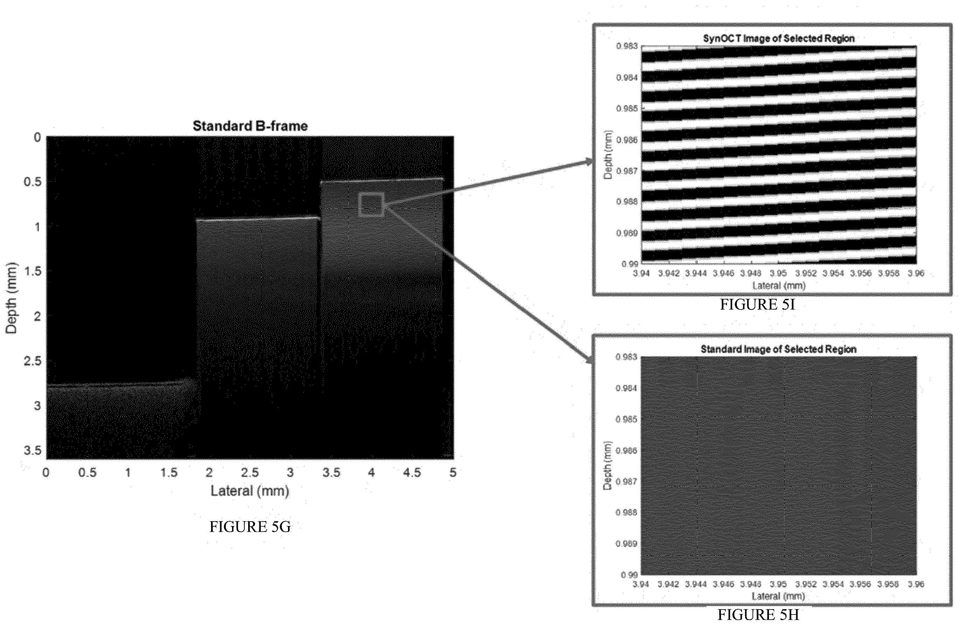

Figure 5G shows a reconstructed B-frame, and Figures 5H and 5I show a magnified portion of a selected region of the B-frame reconstructed using both conventional image reconstruction and the synOCT method 200 respectively. As can be seen, conventional image reconstruction is unable to resolve the fine spatial structure of the object shown in Figure 5G, while the synOCT method 200 is able to resolve the periodic structure of the object.

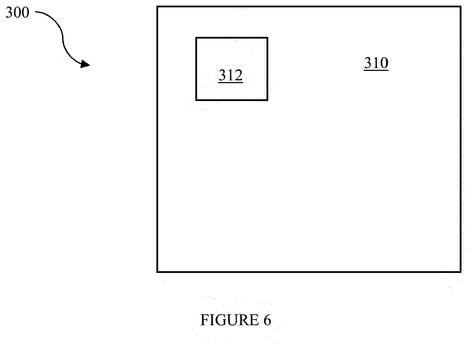

Figure 6 shows an apparatus 300 for imaging an object, in accordance with an embodiment of the invention. In the embodiment shown, the apparatus 300 comprises an imaging system 310 configured to obtain a spectrum of axial spatial frequencies of an object (not shown). In the embodiment shown, the imaging system 310 is an OCT imaging system (such as a SDOCT or SSOCT imaging system), although that is not essential. The apparatus 300 further comprises a processor 312. The processor 312 is configured to generate an image of the object imaged by the imaging system 310. The processor 300 is configured to carry out one or more steps of the methods 100, 200 described above.

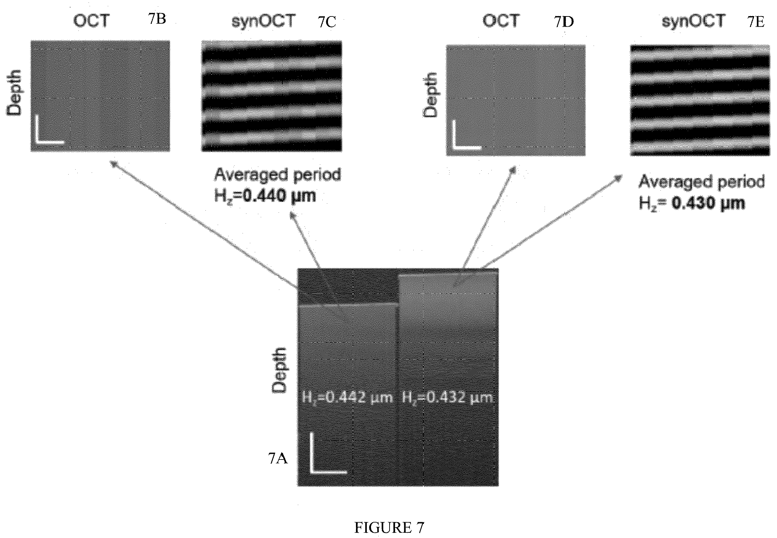

Experimental validation of the methods 100, 200 described above is provided below. Two samples with a well-known internal periodic structure, each sample having a different axial spatial period, were obtained from OptiGrate Corp., USA. The axial spatial periods $H_z$ of the two samples were 431.6nm and 441.7nm respectively, as shown in Figure 7A (scale bar 0.5mm vertical x 0.5mm lateral). The two samples were imaged using a SDOCT TELESTO III system from Thorlabs, Inc. with a central wavelength of 1300nm and a depth resolution of 5.5$\mu$m in air. The spatial periods within the two samples are approx. 8.6 times less than the depth resolution of the SDOCT system used. It is therefore impossible to resolve the period structure of the two samples using conventional OCT image reconstruction, as shown in Figure 7B and 7D (scale bar 0.5$\mu$m vertical x 2$\mu$m lateral). Figures 7B and 7D show magnified portions of an image of each respective sample, reconstructed using conventional OCT image reconstruction.

However, application of the synOCT methods 100, 200 described above - in particular, producing a synthetic spectrum comprising the axial spatial frequencies of the samples (measured using OCT) and corresponding negative frequencies, and reconstructing an image of the depth profile using the synthetic spectrum - allows the fine periodic structure within both samples to be resolved, as shown in Figures 7C and 7E (scale bar 0.5$\mu$m vertical x 2$\mu$m lateral). Measured spatial periods averaged within selected areas of the two samples are as follows:

$H_{z1}$ = 440.25nm (standard deviation 0.077nm), as shown in Figure 7C;
$H_{z2}$ = 430.9nm (standard deviation 0.058nm), as shown in Figure 7E.

The difference between the periods of the two samples, which is approx. 10nm, can be clearly detected using the present invention. It is not possible to resolve such structures and perform quantitative characterization at a given spectral bandwidth using conventional optical techniques.

The super-resolution imaging described above can be realized within the bandwidth of the spatial frequencies collected using a given OCT system, which in turn is dependent on the spectral bandwidth of the light source used. For objects having spatial frequencies within the collected bandwidth of spatial frequencies of a given OCT system, the depth resolution R of an image reconstructed using the synOCT approach of the invention may be estimated or approximated as $R \approx \lambda_{min}/2n$, where R is the resolution, $\lambda_{min}$ is the minimum wavelength of the spectrum of the light source used to image the object, and n is the refractive index of the object. In the examples shown in Figures 7A to 7E, the minimum wavelength for the TELESTO III OCT imaging system was $\lambda_{min}$ = 1177nm, and the refractive index for the two samples was n = 1.483. That provides a depth resolution of approximately 397nm. That depth resolution is in agreement with the spatial periods of the two samples, which are clearly resolved using the synOCT approach of the invention (as shown in Figures 7C and 7E).

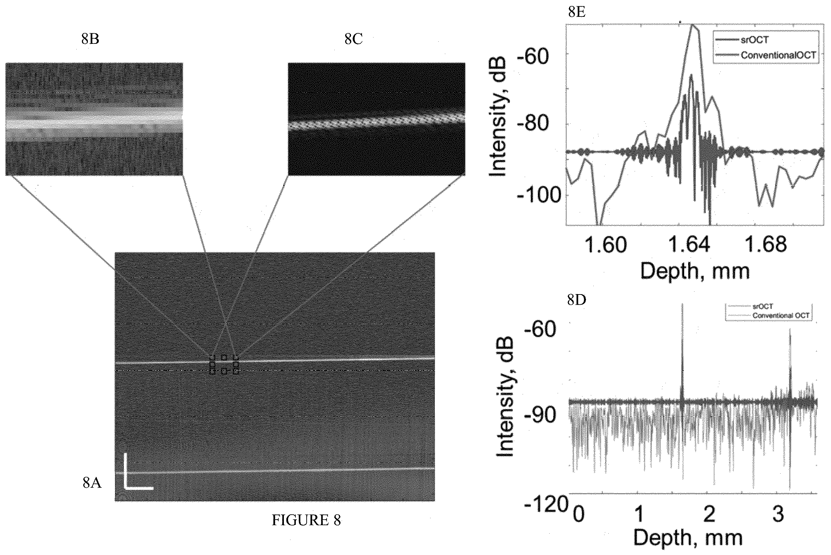

However, for objects having a complicated structure where at least some of the spatial frequencies of the object are not within the directly collected bandwidth, the axial or depth resolution may also be improved using the synOCT approach of the invention. Figure 8A shows a reconstructed image of a glass plate having a thickness of approximately 1mm and a refractive index of 1.52 (scale bar 0.5mm vertical x 0.1mm lateral), with magnified portions of a selected region (corresponding to the blue rectangle), reconstructed using both conventional image reconstruction and the synOCT method 200 shown in Figures 8B and 8C respectively. The improvement in axial resolution using the synOCT approach is clearly demonstrated. Similarly, Figure 8D shows the A-line or A-scan, for both conventional OCT (shown using a red line) and synOCT (shown using a blue line). Figure 8E shows a magnified portion of a selected region of the A-scan of Figure 8D corresponding to the first surface of the glass plate which represents the point spread functions (PSF). The magnified portion shown in Figure 8E illustrates an approx. two times improvement in resolution.

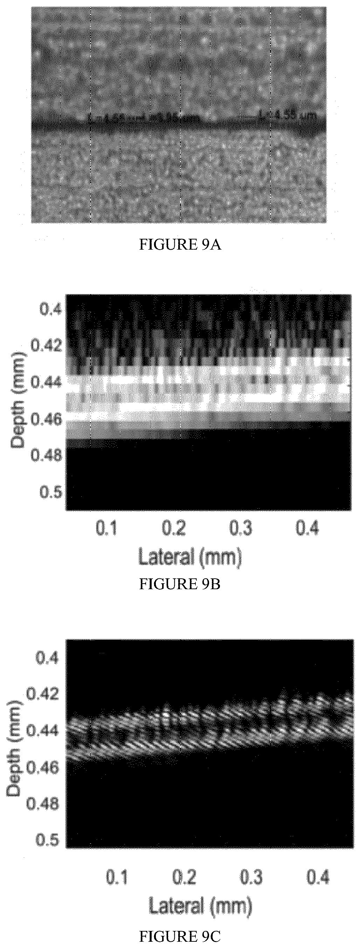

For further comparison, Figures 9A to 9C respectively show an optical microscopy image (objective 10x, NA=0.3), a conventional OCT image and a synOCT image of an air-gap between two microscope slides. The size of the gap was approx. 4$\mu$m, well below the resolution limit of the TELESTO III OCT system used. However, as shown in Figure 9C, the gap between the two glass surfaces can be clearly resolved using the synOCT approach.

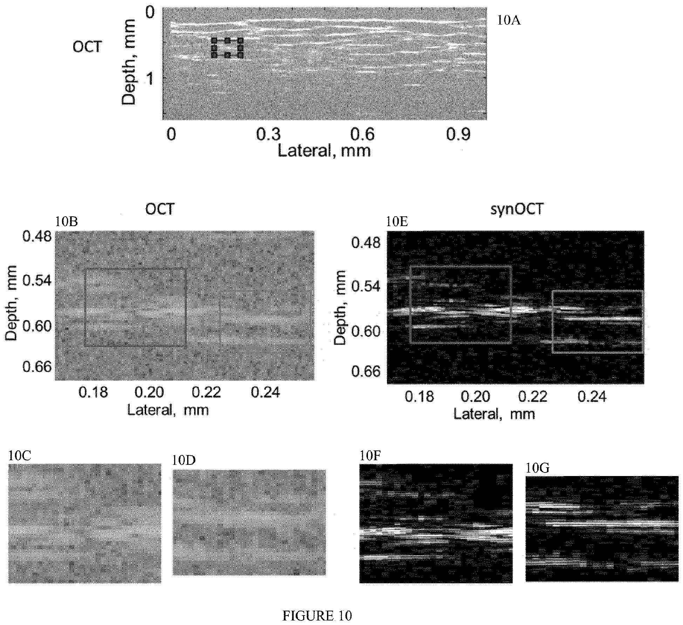

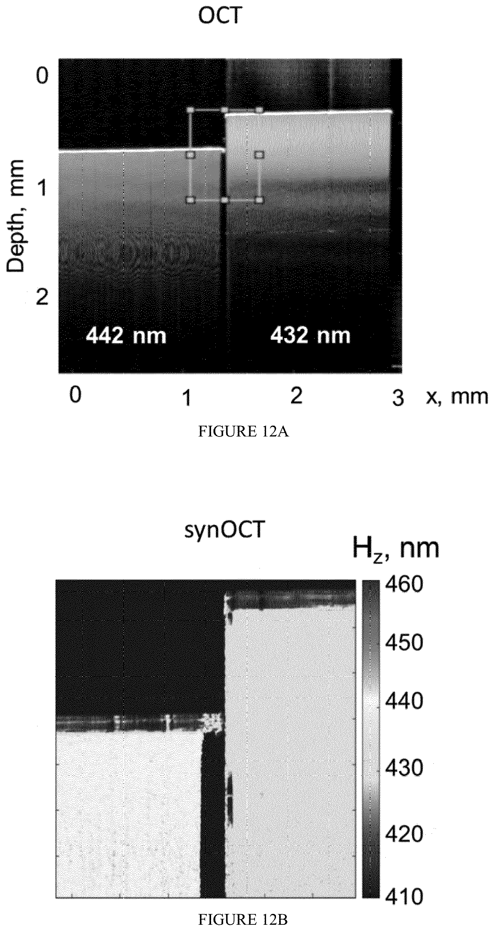

Figure 10A shows a conventional OCT image reconstruction or B-scan of a cucumber. Figure 10B shows a magnified region of the OCT image of Figure 12A (corresponding to the blue rectangle). Figures 10C and 10D show further magnified regions of the magnified OCT image of Figure 10B (corresponding to the red rectangle and green rectangle respectively). In the conventional OCT images, the fine internal structure of the cucumber cannot be visualised, no matter the extent of magnification.

Figure 10E shows a corresponding synOCT image reconstruction of the same magnified portion of Figure 10A (corresponding to the blue rectangle). Figures 10F and 10G show synOCT image reconstructions of the same further magnified regions as shown in Figure 10B (corresponding to the red rectangle and green rectangle respectively in Figure 10E).

The synOCT images of Figures 10E, 10F and 10G demonstrate a clear improvement in depth resolution compared with the corresponding conventional OCT images of Figures 10B, 10C and 10D. Some layers of the cucumber which cannot be seen separately in the conventional OCT images are resolved in the synOCT images. Both the synOCT images and the conventional OCT images were obtained using the same TELESTO III OCT imaging system and formed from the same detected spectral bandwidth.

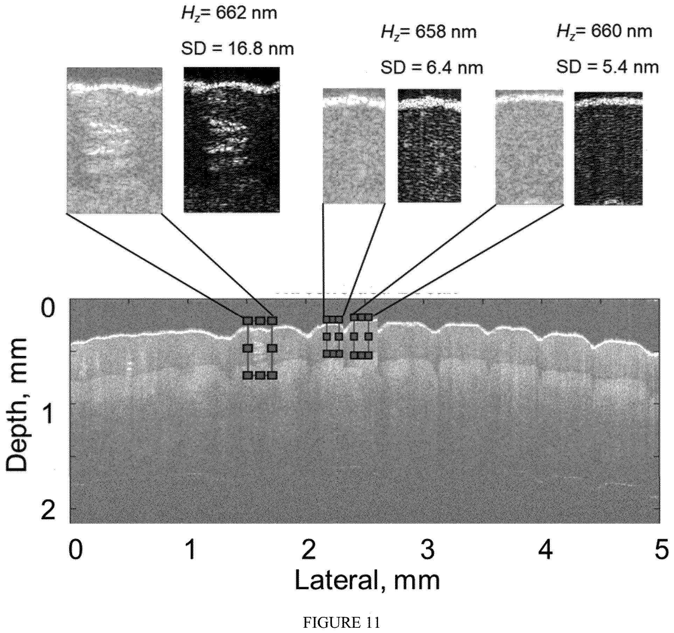

Besides an improvement in resolution and image quality, the synOCT approach of the present invention can provide quantitative information about fine spatial structure of an object, which cannot be resolved using conventional OCT. Figure 11 shows an image of a human finger in vivo, with three magnified portions. For each magnified portion, a conventional OCT image reconstruction and a synOCT image reconstruction is shown. In the magnified synOCT images shown, averaged sizes of the dominant axial spatial periods are calculated, which cannot be calculated for the conventional OCT images for the corresponding magnified portions. That calculation may allow areas with different structures within an object to be identified. For example, an area of the finger comprising a sweat duct (corresponding to the left-most magnified section) comprises structures of different sizes. The standard deviation of the dominant axial spatial periods for this part of the image (16.8nm) is around three times larger than for other areas with more uniform structures, such as the middle and right-most magnified sections which have standard deviations of 6.4nm and 5.4nm respectively. The standard deviations in each of the magnified portions is also substantially 100 times larger than for the synOCT images in Figure 7 described above, illustrating the more complex structure of human skin.

The synOCT approach means the axial spatial period of each resolved structure can be measured from the synOCT image. The object may therefore alternatively be visualised as a colour map or grayscale map of the spatial period distribution of the object, similar to a so-called "nano-sensitive OCT" approach [12-20]. However, in contrast to the nanoscale OCT approach, the improved resolution of the synOCT approach permits visualization of the sub-wavelength structure of an object within an area having a size smaller than the resolution limit of the imaging system used, as described above. As a result, a spatial resolution may be increased more than 20 times relative to typical nanoscale OCT colour map imaging, which has a depth resolution limited to micron scale (substantially $20\mu m$ or greater).

A first approach to form a colour map of the spatial period distribution of the object is as follows. The axial spatial periods are measured directly from the synOCT image for one or more selected areas of the image, as described above. Each of the points or pixels of the synOCT image is then displayed in a colour or shade corresponding to the measured axial spatial period at that point or pixel in the image, or averaged axial spatial period for a plurality of neighbouring pixels, to form a colour map of the spatial period distribution of the object. The colour map approach described above may enable depth-resolved visualization and quantitative measurement or estimation of sub-wavelength internal structure of an object, with both nano-scale sensitivity and dramatically improved depth resolution which is below the resolution limit of the imaging system (e.g., OCT system) used.

A second, alternative approach to form a colour map of the spatial period distribution of the object is as follows (similar to nanosensitive OCT colour map imaging). The synthetic spectrum produced as described above may be decomposed into several zones/volumes, where each zone corresponds to approximately one spatial frequency. Depth resolved images, which correspond to limited bandwidths of spatial frequencies, are formed for each zone by inverse Fourier transform. A profile of the axial spatial frequencies or periods for each small volume inside the object may then be reconstructed by calculated the energy contribution from spatial frequencies for each image. A colour synOCT image can then be formed as a colour map of one or more parameters of the axial spatial frequency profiles. For example, a colour map of the dominant spatial period (period at maximum signal) at a given location may be formed, or a colour map of the mean spatial period at a given location. To visualize nanoscale structural changes, an image may also be formed as a correlation map between axial spatial frequency profiles (which may be compared in space or in time), or an image may be formed as a difference between axial spatial frequency profiles, and so on. Different parameters for visualization can be used depending on what is of interest, for example, relationships between signals at different spatial frequencies, etc. However, as described above, in contrast to the nanoscale OCT approach, the improved resolution of the synOCT approach permits visualization of the sub-wavelength structure of an object within an area having a size smaller than the resolution limit of the imaging system used, significantly improving a depth resolution of the colour synOCT image when compared to nanoscale OCT colour mapping.

Figure 12A shows an OCT image or B-scan of the samples shown in Figure 7 described above. A colour map is generated in accordance with the first approach described above for a selected area of the OCT image (outlined in the blue rectangle in the B-scan) to provide a colour synOCT image of the selected region. The colour synOCT image shown in Figure 12B is a colour map of the dominant axial spatial period of the object. It can be seen that structure of the selected areas within the samples is visualized using colour, and differences in structural size of 10nm or smaller (for example, as small as a few nm) can be clearly displayed (for example, as shown by the colour difference between the two samples in the colour synOCT of Figure 12B). The substantially uniform colour of the two samples in the colour synOCT image demonstrates the substantially uniform periodic structure of the samples (which have axial spatial periods of 441.7nm and 431.6nm respectively). The depth resolution of the colour synOCT images in Figure 12B is $2\mu m$, substantially three times smaller than the resolution limit of the TELESTO III OCT system used. In addition, within the small $2\mu m$ areas the submicron structure is quantified and visualized by virtue of the colour map.

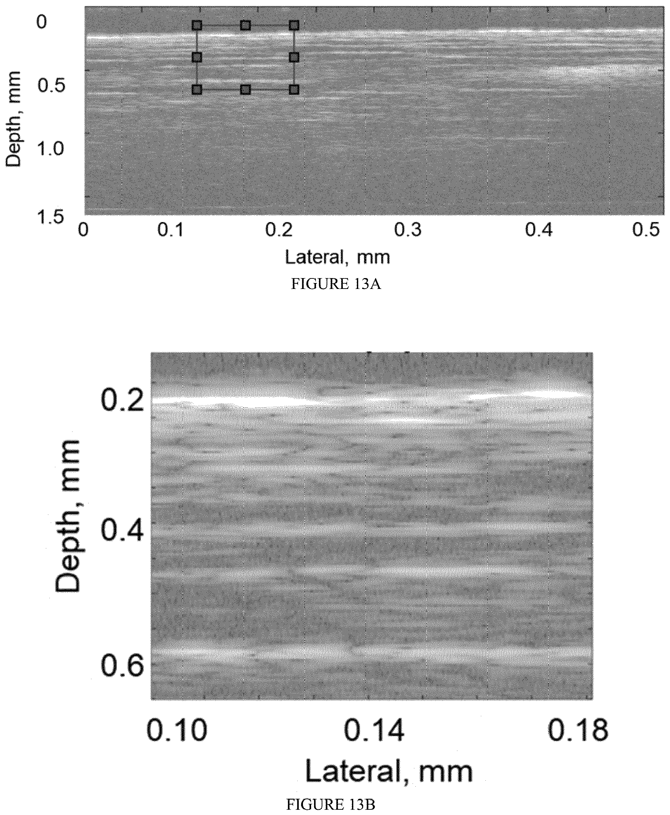

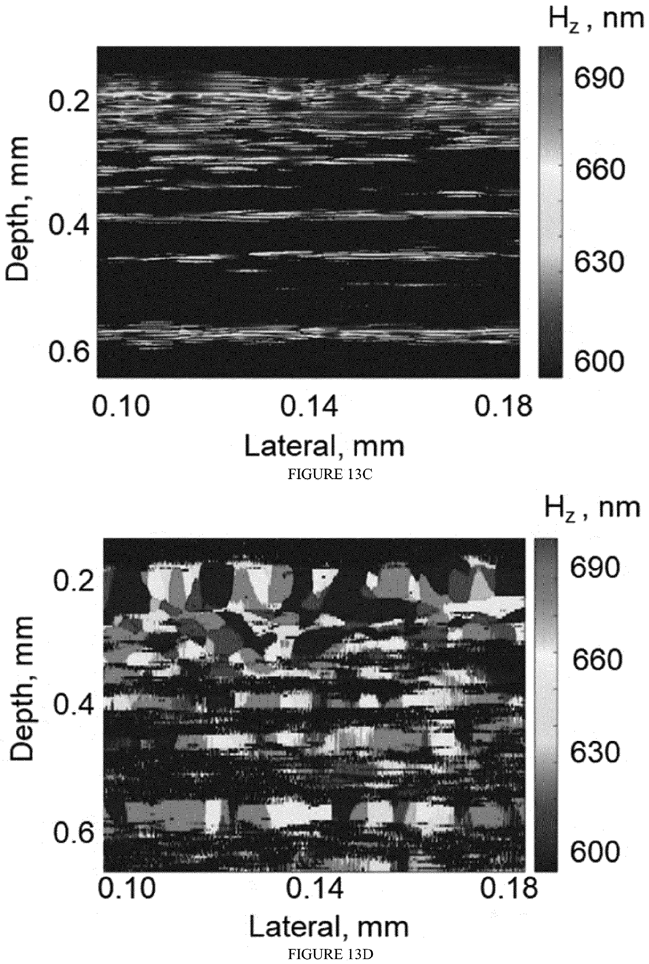

Figure 13A shows a conventional OCT image reconstruction or B-scan of a slice of a potato. Figures 13B, 13C and 13D each show a magnified region of the OCT image of Figure 13A (corresponding to the blue rectangle), using different imaging techniques.

Figure 13B shows a conventional OCT image of the magnified region of the OCT image of Figure 13A. Figure 13C shows a colour synOCT image of the magnified region of the OCT image of Figure 13A. Figure 13D shows a nanosensitive colour map OCT image of the magnified region of the OCT image of Figure 13A. The colour bars in Figures 13C and 13D represent the dominant spatial period of the object in nm.

The size of the areas in which the sub-wavelength structure of the object is visualized in Figure 13C is approximately $2\mu m$, substantially 3 times smaller than the resolution limit of the TELESTO III OCT system used. The significant improvement in resolution in comparison to both the conventional OCT image of Figure 13B and the nanosensitive colour map OCT image of Figure 13D is clearly demonstrated in the colour synOCT image of Figure 13C. The colour synOCT image of Figure 13C allows quantitative information about the sub-wavelength structure to be visualized in an area smaller the resolution limit of the imaging system with nanoscale sensitivity to structural changes, which is simply not possible using nanosensitive OCT imaging.

The synOCT approach and results described above show the potential of the synOCT approach of the present invention. The synOCT approach provides super-resolution using just a single frame. To resolve sub-micron structures within an object using a conventional OCT with a 1300nm central wavelength (as in the TELESTO III OCT system) requires a spectral bandwidth larger than 1150nm which is not currently possible. Moreover, even with such a large spectral bandwidth, it would not be possible to measures the sizes of periods with the nanoscale accuracy described above for the synOCT approach (for example, detecting the difference between periods of 10nm or less).

The colour synOCT imaging approach and results described above also show the potential of colour synOCT imaging for numerous applications, particularly in medicine and biology. Colour synOCT imaging permits depth-resolved visualization of the sub-wavelength internal structure of an object, with nanoscale sensitivity to structural changes and a depth resolution below the resolution limit of the imaging system (e.g., OCT system) used.

From reading the present disclosure, other variations and modifications will be apparent to the skilled person. Such variations and modifications may involve equivalent and other features which are already known in the art of imaging technology, in particular OCT imaging technology, and which may be used instead of, or in addition to, features already described herein.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality, a single processor or other unit may fulfil the functions of several means recited in the claims and any reference signs in the claims shall not be construed as limiting the scope of the claims.

REFERENCES

[1] W. Drexler, U. Morgner, R. K. Ghanta, F. X. Kartner, J. S. Schuman and J. G. Fujimoto, Nat. Med. 7 (4), 502-507 (2001).
[2] J. G. Fujimoto, Nat. Biotechnol 21 (11), 1361-1367 (2003).
[3] D. Cui, X. Liu, J. Zhang, X. Yu, S. Ding, Y. Luo, J. Gu, P. Shum and L. Liu, Opt. Lett. 39 (23), 6727-6730 (2014).
[4] M. Kulkarni, C. Thomas, and J. Izatt, Electron. Lett. 33 (16), 1365-1367 (1997).
[5] E. Bousi and C. Pitris, J Biomed Opt 17 (7), 071307 (2012).
[6] C. Liu, A. Wong, K. Bizheva, P. Fieguth and H. Bie, Opt Express 20 (9), 10200-10211 (2012).
[7] A. Boroomand, A. Wong, E. Li, D. S. Cho, B. Ni and K. Bizheva, Biomed Opt Express 4 (10), 2032-2050 (2013).
[8] X. Liu, S. Chen, D. Cui, X. Yu and L. Liu, Opt Express 23 (20), 26521-26532 (2015).
[9] J. de Wit, K. Angelopoulos, J. Kalkman and G. O. Glentis, Opt. Express 29 (24), 39946-39966 (2021).
[10] P. G. Daneshmand, H. Rabbani and A. Mehridehnavi, IEEE Trans Image Process (2020).
[11] Y. Huang, Z. Lu, Z. Shao, M. Ran, J. Zhou, L. Fang and Y. Zhang, Opt Express 27 (9), 12289-12307 (2019).

[12] S. Alexandrov, H. M. Subhash, A. Zam and M. Leahy, Nanoscale (2014).

[13] S. Alexandrov, P. M. McNamara, N. Das, Y. Zhou, G. Lynch, J. Hogan and M. Leahy, Appl. Phys. Lett. 115 (12) (2019).

[14] N. Das, S. Alexandrov, Y. Zhou, K. E. Gilligan, R. M. Dwyer, M. Leahy, Nanoscale Advances, DOI: 10.1039/D0NA00371A (2020).

[15] N. Das, S. Alexandrov, K. E. Gilligan, R. M. Dwyer, R. B. Saager, N. Ghosh, M. Leahy, J. Biomed. Opt. v.26, N1, 016003, doi: 10.1117/1.JBO.26.1.016003 (2021).

[16] C. Lal, S. Alexandrov, S. Rani, Y. Zhou, T. Ritter, M. Leahy, Biomed. Opt. Express 11, (5) DOI: https://doi.org/10.1364/BOE.389342 (2020).

[17] Y. Zhou, S. Alexandrov, A. Nolan, N. Das, R. Dey and M. Leahy, J. Biophotonics 13 (6) (2020).

[18] Alexandrov, S., Subhash, H. M., Zam, A., and Leahy, M., Patent US 20160238370 A1, (2014). Issued July 3, 2018, and European Patent EP3055643 B1, (2014). Issued July 31, 2019.

[19] S. Alexandrov, A. Arangath, Y. Zhou, M. Murphy, N. Duffy, K. Neuhaus, G. Shaw, R. McAuley, and M. Leahy, Sci. Rep., 11, 17123 (2021).

[20] Dsouza, R. et al. Sci. Rep. 8, doi: ARTN 8777 10.1038/s41598-018-26514-1 (2018).

[21] S. Alexandrov, T. Hillman, T. Gutzler and D. Sampson, Phys. Rev. Lett. 97 (16), 168102 (2006).

[22] T. R. Hillman, T. Gutzler, S. A. Alexandrov and D. D. Sampson, Opt. Express 17 (10), 7873-7892 (2009).

[23] Y. Choi, M. Kim, C. Yoon, T. D. Yang, K. J. Lee and W. Choi, Opt. Lett. 36 (21), 4263-4265 (2011).

[24] V. Mico, Z. Zalevsky, C. Ferreira and J. Garcia, Opt. Express 16 (23), 19260-19270 (2008).

[25] T. Gutzler, T. R. Hillman, S. A. Alexandrov and D. D. Sampson, Opt. Lett. 35 (8), 1136-1138 (2010).

[26] T. S. Ralston, D. L. Marks, P. S. Carney and S. A. Boppart, Nat Phys 3 (2), 129-134 (2007).

[27] J. H. Mason, M. E. Davies and P. O. Bagnaninchi, Opt Express 28 (3), 3879-3894 (2020).

[28] C. J. R. Sheppard, M. Roy and M. D. Sharma, Appl Optics 43 (7), 1493-1502 (2004).

**Claims**

1. An apparatus for imaging an object, the apparatus comprising:

   an imaging system configured to obtain a spectrum comprising one or more axial spatial frequencies representing at least a part of the object; and
   a processor configured to:

   synthesize or generate a spatial harmonic corresponding to each spatial frequency in the spectrum; and
   reconstruct an image of a depth profile of the at least a part of the object using the spatial harmonics.

2. The apparatus of claim 1, wherein the processor is configured to reconstruct the image of the depth profile of the at least a part of the object by superposition of the spatial harmonics.

3. The apparatus of claim 1 or of claim 2, wherein the processor is configured to:

   produce a synthetic spectrum comprising the axial spatial frequencies and a corresponding negative frequency for each of the axial spatial frequencies; and
   reconstruct the image of the depth profile of the at least a part of the object from the synthetic spectrum.

4. The apparatus of any preceding claim, wherein the processor is configured to reconstruct the image of the depth profile of the at least a part of the object having a depth resolution smaller than a resolution limit of the imaging system.

5. The apparatus of any preceding claim, wherein the imaging system is or comprises an optical coherence tomography or optical coherence microscopy imaging system.

6. The apparatus of claim 3 or of any claim dependent directly or indirectly from claim 3, wherein the processor is configured to reconstruct the image of the depth profile of the at least a part of the object by performing an inverse Fourier transform of the synthetic spectrum.

7. The apparatus of claim 3 or of any claim dependent directly or indirectly from claim 3, wherein the processor is configured to represent the synthetic spectrum along an axial direction of the object as:

$$U(v_Z) = \begin{cases} U_1(|v_Z|), for \ v_{Zmin} \leq v_z \leq v_{Zmax}, -v_{Zmax} \leq v_z \leq -v_{Zmin} \\ I_0, for \ v_z = 0 \\ 0, for \ the \ rest \end{cases} .$$

**8.** The apparatus of claim 3 or of any claim dependent directly or indirectly from claim 3, wherein:

the imaging system is configured to obtain a spectrum comprising the one or more axial spatial frequencies and one or more lateral spatial frequencies of the object; and
the processor is configured to produce a synthetic spectrum comprising the axial spatial frequencies and the lateral spatial frequencies, and corresponding negative frequencies for each of the axial spatial frequencies and lateral spatial frequencies.

**9.** The apparatus of any preceding claim, wherein the processor is configured to determine quantitative information about the object from:

the reconstructed image; and/or
the synthetic spectrum.

**10.** The apparatus of claim 9, wherein the processor is configured to generate a map showing a distribution of the determined quantitative information about the object; and optionally

wherein the processor is configured to generate the map by displaying each point in the reconstructed image using a parameter value corresponding to the quantitative information for that point in the reconstructed image; and/or.
wherein the processor is configured to generate the map having a depth resolution smaller than a resolution limit of the imaging system.

**11.** A method of imaging an object, comprising:

obtaining a spectrum comprising one or more axial spatial frequencies representing at least a part of the object;
synthesizing or generating a spatial harmonic corresponding to each spatial frequency in the spectrum; and
reconstructing an image of a depth profile of the at least a part of the object using the spatial harmonics.

**12.** The method of claim 11, comprising reconstructing the image of the depth profile of the at least a part of the object by superposition of the spatial harmonics.

**13.** The method of claim 11 or of claim 12, wherein synthesizing or generating the spatial harmonics and reconstructing the image of the depth profile of the at least a part of the object comprises:

producing a synthetic spectrum comprising the axial spatial frequencies and a corresponding negative frequency for each of the axial spatial frequencies; and
reconstructing an image of a depth profile of the at least a part of the object from the synthetic spectrum.

**14.** A non-transitory computer program comprising instructions which, when the program is executed by a processor, cause the processor to carry out the method of any of claims 11 to 13.

**15.** A computer-readable medium having the computer program of claim 14 stored thereon.

1A

OCT Illumination

$H$

object

$Z$

1B

$I$

0

OCT interferogram

$V_{min}$  $V_{max}$  $V$

Inverse Fourier transform

1C

$I$

$Z$

A-line

1D

$Z$

B-frame

FIGURE 1

FIGURE 2

100 ⌐

| 102 | Obtain spectrum of axial spatial frequencies for object |

| 104 | Produce synthetic spectrum including corresponding negative frequencies |

| 106 | Reconstruct image of object from synthetic spectrum |

FIGURE 3

200 ⌐

| 202 | Obtain interferogram using Fourier domain OCT |

| 204 | Pre-processing interferogram |

| 206 | Produce synthetic spectrum using interferogram and conjugate |

| 208a | IFFT of synthetic spectrum |

| 208b | Repeat for each synthetic spectrum to reconstruct image |

FIGURE 4

FIGURE 5A

FIGURE 5B

FIGURE 5C

FIGURE 5D

FIGURE 5E

FIGURE 5F

Standard B-frame

FIGURE 5G

SynOCT Image of Selected Region

FIGURE 5I

Standard Image of Selected Region

FIGURE 5H

300

312

310

FIGURE 6

FIGURE 7

FIGURE 8

FIGURE 9A

FIGURE 9B

FIGURE 9C

OCT

synOCT

FIGURE 10

FIGURE 11

OCT

FIGURE 12A

synOCT

FIGURE 12B

FIGURE 13A

FIGURE 13B

FIGURE 13C

FIGURE 13D

Europäisches
Patentamt

European
Patent Office

Office européen
des brevets

# EUROPEAN SEARCH REPORT

Application Number

EP 22 16 1221

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAMES D MANTON: "Answering some questions about structured illumination microscopy", ARXIV.ORG, CORNELL UNIVERSITY LIBRARY, 201 OLIN LIBRARY CORNELL UNIVERSITY ITHACA, NY 14853, 16 November 2021 (2021-11-16), XP091087072, | 1,2,4, 9-12,14, 15 | INV. G01B9/02091 |
| A | * the whole document * | 3,5-8,13 | |
| X | ALEXANDROV SERGEY ET AL: "Accessing depth-resolved high spatial frequency content from the optical coherence tomography signal", SCIENTIFIC REPORTS, [Online] vol. 11, no. 1, 1 December 2021 (2021-12-01), XP055951064, DOI: 10.1038/s41598-021-96619-7 Retrieved from the Internet: URL:https://www.nature.com/articles/s41598-021-96619-7.pdf> [retrieved on 2022-08-25] | 1,2,4,5, 9-12,14, 15 | |
| A | * the whole document * | 3,6-8,13 | **TECHNICAL FIELDS SEARCHED (IPC)**<br><br>G01B<br>A61B<br>G01N |
| A,D | US 2016/238370 A1 (ALEXANDROV SERGEY [IE] ET AL) 18 August 2016 (2016-08-18) * the whole document * | 1-15 | |

The present search report has been drawn up for all claims

| Place of search | Date of completion of the search | Examiner |
|---|---|---|
| Munich | 26 August 2022 | Ardelt, Per-Lennart |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 16 1221

This annex lists the patent family members relating to the patent documents cited in the above-mentioned European search report.
The members are as contained in the European Patent Office EDP file on
The European Patent Office is in no way liable for these particulars which are merely given for the purpose of information.

26-08-2022

| Patent document cited in search report | Publication date | Patent family member(s) | | Publication date |
|---|---|---|---|---|
| US 2016238370 A1 | 18-08-2016 | EP | 3055643 A1 | 17-08-2016 |
| | | US | 2016238370 A1 | 18-08-2016 |
| | | WO | 2015052232 A1 | 16-04-2015 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160238370 A1, Alexandrov, S., Subhash, H. M., Zam, A., and Leahy, M.

- EP 3055643 B1

**Non-patent literature cited in the description**

- **W. DREXLER ; U. MORGNER ; R. K. GHANTA ; F. X. KARTNER ; J. S. SCHUMAN ; J. G. FUJIMOTO.** *Nat. Med.,* 2001, vol. 7 (4), 502-507
- **J. G. FUJIMOTO.** *Nat. Biotechnol,* 2003, vol. 21 (11), 1361-1367
- **D. CUI ; X. LIU ; J. ZHANG ; X. YU ; S. DING ; Y. LUO ; J. GU ; P. SHUM ; L. LIU.** *Opt. Lett.,* 2014, vol. 39 (23), 6727-6730
- **M. KULKARNI ; C. THOMAS ; J. IZATT.** *Electron. Lett.,* 1997, vol. 33 (16), 1365-1367
- **E. BOUSI ; C. PITRIS.** *J Biomed Opt,* 2012, vol. 17 (7), 071307
- **C. LIU ; A. WONG ; K. BIZHEVA ; P. FIEGUTH ; H. BIE.** *Opt Express,* 2012, vol. 20 (9), 10200-10211
- **A. BOROOMAND ; A. WONG ; E. LI ; D. S. CHO ; B. NI ; K. BIZHEVA.** *Biomed Opt Express,* 2013, vol. 4 (10), 2032-2050
- **X. LIU ; S. CHEN ; D. CUI ; X. YU ; L. LIU.** *Opt Express,* 2015, vol. 23 (20), 26521-26532
- **J. DE WIT ; K. ANGELOPOULOS ; J. KALKMAN ; G. O. GLENTIS.** *Opt. Express,* 2021, vol. 29 (24), 39946-39966
- **P. G. DANESHMAND ; H. RABBANI ; A. MEHRIDEHNAVI.** *IEEE Trans Image Process,* 2020
- **Y. HUANG ; Z. LU ; Z. SHAO ; M. RAN ; J. ZHOU ; L. FANG ; Y. ZHANG.** *Opt Express,* 2019, vol. 27 (9), 12289-12307
- **S. ALEXANDROV ; H. M. SUBHASH ; A. ZAM ; M. LEAHY.** *Nanoscale,* 2014
- **S. ALEXANDROV ; P. M. MCNAMARA ; N. DAS ; Y. ZHOU ; G. LYNCH ; J. HOGAN ; M. LEAHY.** *Appl. Phys. Lett.,* 2019, vol. 115 (12
- **N. DAS ; S. ALEXANDROV ; Y. ZHOU ; K. E. GILLIGAN ; R. M. DWYER ; M. LEAHY.** *Nanoscale Advances,* 2020
- **N. DAS ; S. ALEXANDROV ; K. E. GILLIGAN ; R. M. DWYER ; R. B. SAAGER ; N. GHOSH ; M. LEAHY.** *J. Biomed. Opt.,* 2021, vol. 26 (1), 016003
- **C. LAL ; S. ALEXANDROV ; S. RANI ; Y. ZHOU ; T. RITTER ; M. LEAHY.** *Biomed. Opt. Express,* 2020, vol. 11 (5
- **Y. ZHOU ; S. ALEXANDROV ; A. NOLAN ; N. DAS ; R. DEY ; M. LEAHY.** *J. Biophotonics,* 2020, vol. 13 (6
- **S. ALEXANDROV ; A. ARANGATH ; Y. ZHOU ; M. MURPHY ; N. DUFFY ; K. NEUHAUS ; G. SHAW ; R. MCAULEY ; M. LEAHY.** *Sci. Rep.,* 2021, vol. 11, 17123
- **DSOUZA, R. et al.** *Sci. Rep.,* 2018, vol. 8
- **S. ALEXANDROV ; T. HILLMAN ; T. GUTZLER ; D. SAMPSON.** *Phys. Rev. Lett.,* 2006, vol. 97 (16), 168102
- **T. R. HILLMAN ; T. GUTZLER ; S. A. ALEXANDROV ; D. D. SAMPSON.** *Opt. Express,* 2009, vol. 17 (10), 7873-7892
- **Y. CHOI ; M. KIM ; C. YOON ; T. D. YANG ; K. J. LEE ; W. CHOI.** *Opt. Lett.,* 2011, vol. 36 (21), 4263-4265
- **V. MICO ; Z. ZALEVSKY ; C. FERREIRA ; J. GARCIA.** *Opt. Express,* 2008, vol. 16 (23), 19260-19270
- **T. GUTZLER ; T. R. HILLMAN ; S. A. ALEXANDROV ; D. D. SAMPSON.** *Opt. Lett.,* 2010, vol. 35 (8), 1136-1138
- **T. S. RALSTON ; D. L. MARKS ; P. S. CARNEY ; S. A. BOPPART.** *Nat Phys,* 2007, vol. 3 (2), 129-134
- **J. H. MASON ; P. O. BAGNANINCHI.** M. E. Davies. *Opt Express,* 2020, vol. 28 (3), 3879-3894
- **C. J. R. SHEPPARD ; M. ROY ; M. D. SHARMA.** *Appl Optics,* 2004, vol. 43 (7), 1493-1502